(19) Europäisches Patentamt
European Patent Office
Office européen des brevets

(11) Veröffentlichungsnummer: **0 089 606**
**B1**

(12) # EUROPÄISCHE PATENTSCHRIFT

(45) Veröffentlichungstag der Patentschrift: **29.10.86**

(51) Int. Cl.[4]: **C 12 Q 1/26**, C 12 Q 1/00

(21) Anmeldenummer: **83102556.4**

(22) Anmeldetag: **15.03.83**

(54) **Verfahren und Reagenz zur Bestimmung von Glycerin.**

(30) Priorität: **19.03.82 DE 3210095**

(43) Veröffentlichungstag der Anmeldung: **28.09.83 Patentblatt 83/39**

(45) Bekanntmachung des Hinweises auf die Patenterteilung: **29.10.86 Patentblatt 86/44**

(84) Benannte Vertragsstaaten: **AT BE CH DE FR GB IT LI LU NL SE**

(56) Entgegenhaltungen:
**EP - A - 0 007 058**
**FR - A - 2 387 992**

(73) Patentinhaber: **BOEHRINGER MANNHEIM GMBH, Patentabteilung, Abt. E Sandhofer Strasse 112-132 Postfach 31 01 20, D-6800 Mannheim 31 Waldhof (DE)**

(72) Erfinder: **Röder, Albert, Dr., Bahnhofstrasse 41, D-8124 Seeshaupt (DE)**
Erfinder: **Nägele, Ulrich, Dr., Bahnhofstrasse 6, D-8131 Bernried (DE)**
Erfinder: **Möllering, Hans, Herrestrasse 10, D-8132 Tutzing (DE)**
Erfinder: **Ziegenhorn, Joachim, Dr., Ina-Seidel-Weg 1, D-8130 Starnberg (DE)**

(74) Vertreter: **Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Postfach 860820, D-8000 München 86 (DE)**

EP 0 089 606 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur enzymatischen Bestimmung von Glycerin und ein Reagenz zur Durchführung dieses Verfahrens.

Die Bestimmung von Triglyceriden (= Glycerinester von langkettigen Fettsäuren) hat für die medizinische Diagnostik eine erhebliche Bedeutung. Ein erhöhter Triglyceridspiegel im Blut ist ein wesentlicher Risikofaktor der Arteriosklerose. Bei hohen Triglyceridwerten, also Hypertriglyceridämie, kommen Koronarinsuffizienz und Herzinfarkt häufiger vor als bei niedrigen Triglyceridwerten. Hypertriglyceridämie begünstigt das Auftreten von Arteriosklerose und Koronarerkrankungen und muss daher frühzeitig erkannt werden, damit die Behandlung rechtzeitig beginnen kann. Eine rasche und zuverlässige durchführbare Methode zur Bestimmung von Triglyceriden bzw. Glycerin hat daher grosse Bedeutung.

Die bekannten und brauchbaren Methoden zur Triglyceridbestimmung basieren auf der enzymatischen Hydrolyse der Triglyceride mittels Lipase/Esterase und Bestimmung des freigesetzten Glycerins mittels Glycerokinase/Pyruvatkinase/Lactatdehydrogenase (A.W. Wahlefeld, in Methods of Enzymatic Analysis IV, H.U. Bergmeyer, Ed. Academic Press, New York, London, 1974, S. 1831–1835).

Dieses bekannte Verfahren weist jedoch wesentliche Nachteile auf. Um Störungen durch Eigenfärbungen des Serums (Bilirubin, Hämoglobin) sowie Trübungen zu eliminieren, müssen stets Probenleerwert-Bestimmungen durchgeführt werden, was den Zeitbedarf pro Analyse sowie den Reagenzverbrauch und damit die Kosten erhöht. Ferner ergeben sich infolge der Anwesenheit zahlreicher empfindlicher Coenzyme und Enzyme nur relativ geringe Haltbarkeiten für die Gebrauchslösungen.

Neben diesem UV-Test sind auch Farbtests auf Basis einer Formazan-Bildung bekannt. Letztere erlauben zwar die Bestimmung gegen einen Reagenzien-Leerwert, sind jedoch relativ kompliziert aufgebaut und störanfällig.

Bekannt sind weiter Methoden und Reagenzien, bei welchen Glycerin mittels Glycerindehydrogenase (DE-PS 2 831 580) oder Glycerinoxidase (DE-AS 2 817 087) bestimmt wird. Durch diese Methoden werden zwar verschiedene Nachteile der früher bekannten Verfahren beseitigt, jedoch wäre eine weitere Verbesserung hinsichtlich Empfindlichkeit und Spezifität der Bestimmungsmethode erwünscht.

Es besteht daher ein Bedarf an einem Verfahren und einem Reagenz, welches die oben geschilderten Nachteile nicht aufweist. Insbesondere besteht ein Bedarf an einem Verfahren, welches mit einem einfach in guter Ausbeute zugänglichen Enzym durchgeführt werden kann und welches eine verbesserte Empfindlichkeit und Spezifität liefert.

Gelöst wird diese Aufgabe erfindungsgemäss durch ein Verfahren zur Bestimmung von Glycerin, welches darin besteht, dass Glycerin in wässrigem Medium in Gegenwart von Sauerstoff mit Glycerindehydrogenase, NAD und Glycerinoxidase inkubiert und entweder der Sauerstoffverbrauch oder gebildetes $H_2O_2$ bestimmt werden.

Erfindungsgemäss wird überraschenderweise eine erhöhte Spezifität und Empfindlichkeit der Bestimmung erzielt. Der Reaktionsmechanismus ist zwar nicht bekannt, es wird jedoch angenommen, dass primär durch die Glycerindehydrogenase Dihydroxyaceton gebildet wird, welches von der Glycerinoxidase unter Sauerstoffverbrauch und Bildung von $H_2O_2$ oxidiert wird.

Gemäss der Erfindung erfolgt die Bestimmung mit Glycerinoxidase (Glyc-OD) in Gegenwart von Glycerindehydrogenase und ihrem Coenzym NAD. Glycerindehydrogenase EC 1.1.1.6 und Glycerinoxidase sind bekannt und beispielsweise aus Mikroorganismen erhältlich bzw. handelsüblich. Es wurde gefunden, dass in Gegenwart von Glycerindehydrogenase (Glyc-DH) die Empfindlichkeit und Spezifität der Bestimmung mit Glycerinoxidase weiter verbessert wird.

Das Verfahren der Erfindung kann auch bei vorheriger oder gleichzeitiger enzymatischer Verseifung von Triglyceriden unter Freisetzung von Glycerin oder bei vorheriger chemischer Verseifung von Triglyceriden durchgeführt werden. Zur chemischen Verseifung eignet sich beispielsweise alkoholische Kalilauge, zur enzymatischen Verseifung Lipase mit Esterase oder Esterase allein.

Die Messung des Sauerstoffverbrauchs oder der $H_2O_2$-Bildung kann nach den hierfür bekannten Methoden erfolgen.

Geeignete Methoden zur Bestimmung des Sauerstoffverbrauchs sind beispielsweise Gaschromatographie und elektrochemische Bestimmungsverfahren. Bevorzugt wird die polarographische Bestimmung mittels Sauerstoffelektrode, da sich dieses Verfahren besonders zur automatischen Durchführung der Bestimmung eignet. Derartige Bestimmungsmethoden sind bekannt. Besonders geeignet erwiesen sich die in den deutschen Offenlegungsschriften 2 130 340 und 2 130 308 beschriebenen Methoden zur polarographischen Messung des Sauerstoffverbrauchs in wässrigen Medien. Gebildetes Wasserstoffperoxid kann sowohl titrimetrisch als auch potentiometrisch, polarographisch und colorimetrisch sowie enzymatisch bestimmt werden. Bevorzugt werden die enzymatischen Methoden unter Verwendung von Katalase oder Peroxidase, da diese nicht nur äusserst spezifisch und zuverlässig sind, sondern auch mit der Hauptreaktion unter Bildung von Wasserstoffperoxid auf einfachste Weise kombiniert werden können. Als geeignet erwies sich auch die Bestimmung mittels Katalase in Gegenwart von β-Diketonen, z.B. Acetylaceton und Methanol bzw. Äthanol oder Methylenglykol, sowie die Bestimmung mittels Peroxidase in Gegenwart eines oder mehrerer Chromogene. Bei der Bestimmung mittels Katalase, Acetylaceton und Methanol wird letzteres zu Formaldehyd oxidiert, der mit Acetylaceton eine Farbreaktion eingeht, die gemessen werden kann. Bei der Bestimmung mittels Peroxidase werden als Chromophor Verbindungen eingesetzt, die nach der Reaktion photometrisch bestimmt werden können. Ein Beispiel für ein geeignetes Chromophor ist 2,2'-Aminobenzthiazolinsulfonsäure (ABTS). Ein anderes Beispiel ist das Indikatorsystem nach Trinder (Ann. Clin. Biochem. 6 (1969), 24–27), bei dem Phenol und 4-Aminoantipyrin (4-AAP) in Gegenwart von POD und unter der Einwirkung von $H_2O_2$ oxidativ zu einem Farbstoff gekuppelt werden. Anstelle von Phenol können andere aromatische Alkohole, wie p-Chlorphenol, Anilinderivate, Naphthol, Naphtholderivate, Naphthylamin, Naphthylaminderivate, Aminochinoline, Hydroxychinoline, Dihydroxyphenylessigsäure und ähnlich reagierende Substanzen eingesetzt werden. Anstelle von 4-Aminoantipyrin können 4-Aminoantipyrin-Derivate, wie 4-Aminoantipyrinamid, Phenylendiaminsulfonsäure, MBTH (Methylbenzothiazolonhydrazon) S-MBTH (sulfoniertes Methylbenzothiazolonhydrazon), MBTH- und S-MBTH-Derivate sowie ähnlich reagierende Verbindungen eingesetzt werden.

Als besonders geeignet und daher im Rahmen der Erfindung bevorzugt wird die Umsetzung in Gegenwart von Peroxidase und einem Chromogen, wie p-Chlorphenol/4-Aminoantipyrin oder ABTS. Es wurde hier auch bei kurzen Reaktionszeiten ein linearer Zusammenhang zwischen Extinktionsdifferenz und Glycerinkonzentration festgestellt, so dass sich diese Ausführungsform des erfindungsgemässen Verfahrens besonders zur kinetischen Bestimmung eignet, aber auch zur Endpunktbestimmung verwendbar ist.

NAD kann im Überschuss über die stöchiometrisch benötigte Menge zugesetzt werden, vorzugsweise gibt man jedoch lediglich katalytische Mengen NAD und ein NAD-regenerierendes System zu. Geeignete NAD-regenerierende Systeme sind beispielsweise beschrieben in der DE-OS 2 834 705. Ein bevorzugtes NAD-regenerierendes System zur Verwendung im Rahmen der Erfindung besteht aus Lactatdehydrogenase und ihrem Substrat Pyruvat.

Bei dieser Ausführungsform der Erfindung sollte der pH-Wert zwischen 7,0 und 9,0 eingestellt werden, dann in diesem Bereich sämtliche beteiligten Enzyme, also Glycerinoxidase (Glyc-OD), Glyc-DH und gegebenenfalls Peroxidase (POD) eine für das Verfahren gut geeignete Aktivität aufweisen.

Überraschenderweise verläuft die Umsetzung in dieser Ausführungsform glatt und quantitativ unter Verbrauch von molekularem Sauerstoff und Bildung von $H_2O_2$, obwohl die beiden Enzyme Glyc-OD und Glyc-DH um das Glycerin konkurrieren und die Glyc-DH weder Sauerstoff verbraucht noch $H_2O_2$ bildet.

Durch die kombinierte Anwendung von Glyc-OD und Glyc-DH wird der Empfindlichkeitsbereich mindestens bis 0,001 Mol/l herabgesetzt.

Die Durchführung der Messung kann sowohl nach dem Endwertverfahren gegen einen Reagenzienleerwert oder nach dem kinetischen Verfahren erfolgen.

Auch diese Ausführungsform des erfindungsgemässen Verfahrens kann unter gleichzeitiger enzymatischer Verseifung von Triglyceriden, bei welcher Glycerin freigesetzt und erfindungsgemäss sofort bestimmt wird, durchgeführt werden.

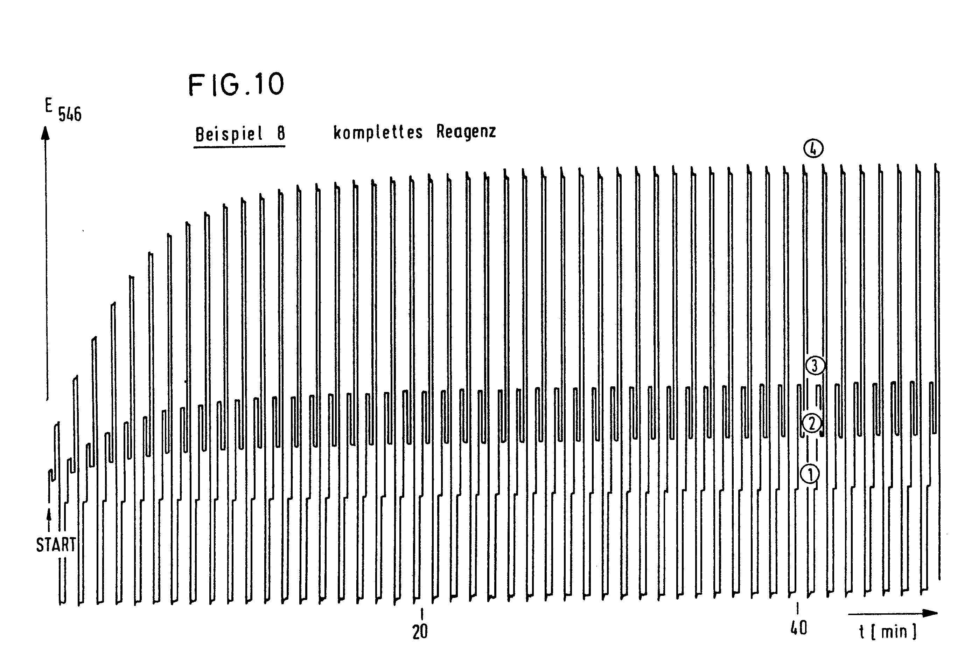

Das Verfahren eignet sich daher besonders auch für Analysenautomaten, da sich durch Eichung mit einem einzigen Standard die Glycerinkonzentration exakt bestimmen lässt. Dies zeigt sich augenfällig in den in der Zeichnung graphisch dargestellten Ergebnissen, die an Analysenautomaten erstellt wurden. Die Figuren 1 bis 10 der Zeichnung werden in den Beispielen näher erläutert.

Gegenstand der Erfindung ist weiter ein Reagenz zur Bestimmung von Glycerin, welches aus Glyc-DH, NAD, Glyc-OD und einem System zur Bestimmung von $H_2O_2$ besteht. In einer ersten bevorzugten Ausführungsform besteht dieses Reagenz im wesentlichen aus Glyc-DH, NAD, Glyc-OD, Peroxidase, wenigstens einem Chromogen und Puffer, einzeln oder gemischt. Als Chromogen wird ABTS bevorzugt. Ein anderes bevorzugtes Farbindikator-System ist das System nach Trinder.

In einer weiteren bevorzugten Ausführungsform besteht das Reagenz im wesentlichen aus Glyc-DH, Glyc-OD, NAD, Katalase, Acetylaceton, Methanol und Puffer, einzeln oder gemischt.

Die oben erwähnten bevorzugten Reagenzkombinationen können ausser den aufgeführten obligaten Bestandteilen zusätzlich übliche Lösungsmittel, Stabilisatoren oder/und oberflächenaktive Substanzen enthalten. Alle diese Zusatzstoffe sind dem Fachmann bekannt und in Nachweissystemen für Wasserstoffperoxid üblich. Vorzugsweise enthalten die erfindungsgemässen Reagenzien zusätzlich noch lipolytische Enzyme, wie Lipase/Esterase oder Esterase allein, so dass sie auch zur Bestimmung von gebundenem Glycerin, welches in Form von Triglyceriden vorliegt, eingesetzt werden können.

In einer bevorzugten Ausführungsform enthält das erfindungsgemässe Reagenz zusätzlich ein NAD-regenerierendes System.

Für die NAD-regenerierenden Systeme gilt das oben zum Verfahren Mitgeteilte in gleicher Weise. Das bevorzugte NAD-regenerierende System besteht aus Lactatdehydrogenase (LDH) und Pyruvat. Die Gegenwart des NAD-regenerierenden Systems macht es möglich, mit einer katalytischen Menge NAD auszukommen.

Vorzugsweise enthält das erfindungsgemässe Reagenz die essentiellen Bestandteile in folgenden Mengenverhältnissen:

| | |
|---|---|
| Glycerinoxidase | 10 bis 200 U/ml |
| Glycerindehydrogenase | 10 bis 200 U/ml |
| NAD | 1 bis 50 mMol/l. |

Falls ein NAD-regenerierendes System zugesetzt wird, besteht es vorzugsweise aus
Lactatdehydrogenase 2 bis 100 U/ml,
Pyruvat 1 bis 50 mMol/l.

Das bevorzugte $H_2O_2$-Nachweissystem besteht aus
Peroxidase 0,5 bis 20 U/ml,
4-Aminoantipyrin 0,2 bis 5 mMol/l,
Phenolderivat 1 bis 100 mMol/l.

Falls Glycerin erst aus Triglyceriden freigesetzt werden soll, kann das Reagenz zusätzlich Lipase und/oder Esterase, vorzugsweise eine Cholesterinesterase enthalten, wobei für die oben angegebene bevorzugte mengenmässige Zusammensetzung des Reagenz in diesem Falle 2 bis 50 U/ml zweckmässig sind.

Die Erfindung schafft ein einfaches Verfahren und Reagenz mit überlegener Spezifität und Empfindlichkeit zur Bestimmung von Glycerin. Das Verfahren ist insbesondere auch für die Anwendung in Automaten geeignet.

Die folgenden Beispiele erläutern die Erfindung weiter:

Beispiel 1
Reagenz:

| Reagenz-Zusammensetzung | | |
|---|---|---|
| Tricin | 100 mMol | pH 8,0 |
| $(NH_4)_2SO_4$ | 160 mMol | |
| $ZnSO_4 \times 7\ H_2O$ | 0,1 mMol | |
| $K_2SO_4$ | 50 mMol | |
| $MgSO_4 \times 7\ H_2O$ | 20 mMol | |
| p-Chlorphenol | 10 mMol | |
| 4-Aminoantipyrinamid | 0,5 mMol | |
| Na-Cholat | 3,5 mMol | |
| Detergens 1 | 0,09% (Dobanol) | |
| Detergens 2 | 0,06% (Genapo X 80) | |
| NAD | 5 mMol | |
| Pyruvat | 5 mMol | |
| Lactat-Dehydrogenase | 10 000 U/l | |
| Cholesterin-Esterase | 8 000 U/l | |
| Glycerin-Dehydrogenase | 30 000 U/l | |
| Glycerinoxidase | 80 000 U/l | |
| Peroxidase | 1 500 U/l | |

Probe:

Wässrige Glycerinlösungen im Bereich von 1 bis 10 mMol/l.

Bestimmungsansatz:

Messstrahlung: 546 nm
Schichtdicke: 1 cm
Temperatur: 25 °C.

1 ml Reagenz in die Küvette geben und mit 0,01 ml Probe starten. Extinktion nach 20 Minuten gegen Reagenzleerwert messen. Für die quantitative Bestimmung wird dieser ΔE über einen Standard zur Glycerin-Konzentration in Beziehung gesetzt. Die Mitführung eines Probenleerwerts ist nicht erforderlich. Eine durch Auftragen der Glycerin-Konzentration gegen die Extinktion bei 546 um und 25 °C erhaltene Kurve ist in Figur 1 der Zeichnung wiedergegeben. Die Extinktionsänderung in Abhängigkeit von der Zeit bei 25 °C ist in Figur 2 der Zeichnung wiedergegeben.

Beispiel 2

Reagenz: Es wird das in Beispiel 1 beschriebene Reagenz verwendet.

Proben: Verschiedene Humanseren bis ca. 450 mg Triglyceride/dl

Bestimmungsansatz:

Messstrahlung 546 nm
Schichtdicke 1 cm
Temperatur 25 °C bis 37 °C.

1,0 ml Reagenz in die Küvette geben und mit 0,01 ml Serum starten. Extinktion nach 25 min gegen Reagenzleerwert messen. Für die quantitative Bestimmung wird dieser ΔE über einen Glycerin-Standard zur Triglycerid-Konzentration in Beziehung gesetzt. Eine durch Auftragen der Triglycerid-Konzentration bei 546 um und 25 °C erhaltenen Kurve ist in Figur 3 wiedergegeben. Die Mitführung eines Probenleerwerts ist nicht erforderlich. Die Extinktionsänderung in Abhängigkeit von der Zeit ist in Figur 4 wiedergegeben.

Beispiel 3 bis 8
Reagenzzusammensetzung:

| | Beispiel 3 und 4 | Beispiel 5 und 6 | Beispiel 7 und 8 |
|---|---|---|---|
| Tricin | 100 mmol/l | 100 mmol/l | 100 mmol |
| $(NH_4)_2SO_4$ | 160 mmol/l | 160 mmol/l | 160 mmol/l |
| $ZnSO_4 \times 7H_2O$ | 0,1 mmol/l | 0,1 mmol/l | 0,1 mmol/l |
| $K_2SO_4$ | 50 mmol/l | 50 mmol/l | 50 mmol/l |
| $MgSO_4 \times 7H_2O$ | 20 mmol/l | 20 mmol/l | 20 mmol/l |
| p-Chlorphenol | 10 mmol/l | 10 mmol/l | 10 mmol/l |
| 4-Aminoantipyrinamid | 0,5 mmol/l | 0,5 mmol/l | 0,5 mmol/l |
| Na-Cholat | 3,5 mmol/l | 3,5 mmol/l | 3,5 mmol/l |
| Dobanol | 0,09 ./. | 0,09 ./. | 0,09 ./. |
| Genapol X-80 | 0,06 ./. | 0,06./. | 0,06./. |
| NAD | – | 5 mmol/l | 5 mmol/l |
| Pyruvat | – | – | 5 mmol/l |
| Lactat-Dehydrogenase | – | – | 10 000 U/l |
| Cholesterin-Esterase | 8 000 U/l | 8 000 U/l | 8 000 U/l |
| Glycerin-Dehydrogenase | – | 30 000 U/l | 30 000 U/l |
| Glycerin-Oxidase | 80 000 U/l | 80 000 U/l | 80 000 U/l |
| Peroxidase | 1 500 U/l | 1 500 U/l | 1 500 U/l |

Proben:

In den Beispielen 3, 5 und 7 wurden Glycerinstandard-Lösungen entsprechend 100, 200 und 500 mg Triglyceride/dl eingesetzt.

In den Beispielen 4, 6 und 8 wurden Humanseren mit ca. 100, 200 und 600 mg Triglyceride/dl eingesetzt.

Bestimmungsansatz:

Wie in Beispiel 2 angegeben.

Die Bestimmungen wurden wie im Beispiel 2 beschrieben, durchgeführt. Die erhaltenen Ergebnisse sind in den Fig. 5 bis 10 wiedergegeben in Form einer Kurvenschaar, die die Extinktionsänderung in Abhängigkeit von der Zeit für die verschiedenen vorstehend angegebenen Triglyceridmengen zeigt. Kurve 1 zeigt dabei in jedem Fall den Reagenzleerwert, die Kurven 2, 3 und 4 werden mit zunehmendem Gehalt an Triglycerid entsprechend den vorstehenden Angaben erhalten.

## Patentansprüche

1. Verfahren zur Bestimmung von Glycerin, dadurch gekennzeichnet, dass Glycerin in wässrigem Medium in Gegenwart von Sauerstoff mit Glycerindehydrogenase, NAD und Glycerinoxidase inkubiert und entweder der Sauerstoffverbrauch oder gebildetes $H_2O_2$ bestimmt werden.

2. Verfahren nach Anspruch 1, dadurch gekennzeichnet, dass der Sauerstoffverbrauch polarographisch gemessen wird.

3. Verfahren nach Anspruch 1, dadurch gekennzeichnet, dass gebildetes $H_2O_2$ enzymatisch mit Katalase oder Peroxidase bestimmt wird.

4. Verfahren nach Anspruch 3, dadurch gekennzeichnet, dass man Peroxidase und ein Chromogen zusetzt und die Färbung bzw. Extinktionsänderung bestimmt.

5. Verfahren nach einem der Ansprüche 1 bis 4, dadurch gekennzeichnet, dass man die Messung kinetisch in einem vorbestimmten kurzen Zeitintervall durchführt.

6. Verfahren nach Anspruch 1, dadurch gekennzeichnet, dass man NAD zusammen mit einem NAD-regenerierenden System zusetzt.

7. Verfahren nach Anspruch 6, dadurch gekennzeichnet, dass man als NAD-regenerierendes System Pyruvat und Lactatdehydrogenase verwendet.

8. Verfahren nach einem der vorhergehenden Ansprüche, dadurch gekennzeichnet, dass man bei pH 7,0 bis 9,0 arbeitet.

9. Reagenz zur Bestimmung von Glycerin, dadurch gekennzeichnet, dass es aus Glyc-DH, NAD, Glyc-OD und einem System zur Bestimmung von $H_2O_2$ besteht.

10. Reagenz nach Anspruch 9, dadurch gekennzeichnet, dass es zusätzlich ein Mittel zur Verseifung von verestertem Glycerin enthält.

11. Reagenz nach Anspruch 9 oder 10, dadurch gekennzeichnet, dass das System zur Bestimmung von $H_2O_2$ aus Peroxidase, wenigstens einem Chromogen und Puffer besteht.

12. Reagenz nach einem der Ansprüche 9 bis 11, dadurch gekennzeichnet, dass es zusätzlich ein NAD-regenerierendes System enthält.

13. Reagenz nach Anspruch 12, dadurch gekennzeichnet, dass das NAD-regenerierende System aus Pyruvat und Lactatdehydrogenase besteht.

14. Reagenz nach Anspruch 11, dadurch gekennzeichnet, dass das Chromogen aus p-Chlorphenol und 4-Aminoantipyrinamid besteht.

15. Reagenz nach Anspruch 14, dadurch gekennzeichnet, dass es 5 bis 15 µMol/ml p-Chlorphenol und 0,1 bis 1,0 µMol/ml 4-Aminoantipyrinamid enthält.

16. Reagenz nach Anspruch 11, dadurch gekennzeichnet, dass das Chromogen aus 2,2′-Azinodi-(3-äthyl-benzthiazolinsulfonsäure-6)-diammoniumsalz besteht.

17. Reagenz nach Anspruch 11, dadurch gekennzeichnet, dass es Glycylglycinpuffer oder Tricinpuffer, pH 7,0 bis 9,0 enthält.

18. Reagenz nach Anspruch 11, dadurch gekennzeichnet, dass es zusätzlich ein oberflächenaktives Mittel enthält.

19. Reagenz nach Anspruch 11, dadurch gekennzeichnet, dass es auf einem Trägermaterial, wie Papier, imprägniert vorliegt.

## Claims

1. Process for the determination of glycerol, characterised in that glycerol is incubated in an aqueous medium in the presence of oxygen with glycerol dehydrogenase, NAD and glycerol oxidase and either the oxygen consumption or the $H_2O_2$ formed is determined.

2. Process according to claim 1, characterised in that the oxygen consumption is measured polarographically.

3. Process according to claim 1, characterised in that $H_2O_2$ formed is determined enzymatically with catalase or peroxidase.

4. Process according to claim 3, characterised in that one adds peroxidase and a chromogen and determines the coloration or extinction change.

5. Process according to one of claims 1 to 4, characterised in that one carries out the measurement kinetically in a predetermined short interval of time.

6. Process according to claim 1, characterised in that one adds NAD, together with an NAD-regenerating system.

7. Process according to claim 6, characterised in that one uses pyruvate and lactate dehydrogenase as NAD-regenerating system.

8. Process according to one of the preceding claims, characterised in that one works at pH 7.0 to 9.0.

9. Reagent for the determination of glycerol, characterised in that it consists of Glyc-DH, NAD, Glyc-OD and a system for the determination of $H_2O_2$.

10. Reagent according to claim 9, characterised in that it additionally contains an agent for the saponification of esterified glycerol.

11. Reagent according to claim 9 or 10, characterised in that the system for the determination of $H_2O_2$ consists of peroxidase, at least one chromogen and buffer.

12. Reagent according to one of claims 9 to 11, characterised in that it additionally contains an NAD-regenerating system.

13. Reagent according to claim 12, characterised in that the NAD-regenerating system consists of pyruvate and lactate dehydrogenase.

14. Reagent according to claim 11, characterised in that the chromogen consists of p-chlorophenol and 4-aminoantipyrinamide.

15. Reagent according to claim 14, characterised in that it contains 5 to 15 µMol/ml. p-chlorophenol and 0.1 to 1.0 µMol/ml. 4-aminoantipyrinamide.

16. Reagent according to claim 11, characterised in that the chromogen consists of 2,2′-azinodi-(3-ethylbenzthiazoline-6-sulphonic acid) diammonium salt.

17. Reagent according to claim 11, characterised in that it contains glycylclycine buffer or tricin buffer, pH of 7.0 to 9.0

18. Reagent according to claim 11, characterised in that it additionally contains a surface-active agent.

19. Reagent according to claim 11, characterised in that it is present impregnated on a carrier material, such as paper.

**Revendications**

1. Procédé de dosage du glycérol, caractérisé en ce que le glycérol est mis à incuber en milieu aqueux en présence d'oxygène avec de la glycéroldéshydrogénase, du NAD et de la glycéroloxydase et en ce qu'on détermine soit la consommation d'oxygène, soit le $H_2O_2$ formé.

2. Procédé suivant la revendication 1, caractérisé en ce que la consommation d'oxygène est mesurée polarographiquement.

3. Procédé suivant la revendication 1, caractérisé en ce que le $H_2O_2$ formé est déterminé enzymatiquement avec de la catalase ou de la peroxydase.

4. Procédé suivant la revendication 3, caractérisé en ce qu'on ajoute de la peroxydase et un chromogène et en ce qu'on détermine la coloration ou la modification de l'extinction.

5. Procédé suivant l'une des revendications 1 à 4, caractérisé en ce qu'on effectue la mesure cinétiquement dans un court intervalle de temps déterminé à l'avance.

6. Procédé suivant la revendication 1, caractérisé en ce qu'on ajoute du NAD en même temps qu'un système régénérant le NAD.

7. Procédé suivant la revendication 6, caractérisé en ce qu'on utilise comme système régénérant le NAD du pyruvate et de la lactatedéshydrogénase.

8. Procédé suivant l'une des revendications précédentes, caractérisé en ce qu'on opère à un pH de 7,0 à 9,0.

9. Réactif pour le dosage du glycérol, caractérisé en ce qu'il se compose de Glyc-DH, NAD, Glyc-OD et d'un système pour le dosage de $H_2O_2$.

10. Réactif suivant la revendication 9, caractérisé en ce qu'il contient en outre un agent pour la saponification du glycérol estérifié.

11. Réactif suivant la revendication 9 ou 10, caractérisé en ce que le système pour le dosage de $H_2O_2$ se compose de peroxydase, d'au moins un chromogène et d'un tampon.

12. Réactif suivant l'une des revendications 9 à 11, caractérisé en ce qu'il contient en outre un système régénérant le NAD.

13. Réactif suivant la revendication 12, caractérisé en ce que le système régénérant le NAD se compose de pyruvate et de lactatedéshydrogénase.

14. Réactif suivant la revendication 11, caractérisé en ce que le chromogène se compose de p-chlorophénol et de 4-aminoantipyrinamide.

15. Réactif suivant la revendication 14, caractérisé en ce qu'il contient 5 à 15 µmoles/ml de p-chlorophénol et 0,1 à 1,0 µmole/ml de 4-aminoantipyrinamide.

16. Réactif suivant la revendication 11, caractérisé en ce que le chromogène se compose du sel de diammonium de l'acide 2,2′-azinodi-(3-éthylbenzothiazolinesulfonique-6).

17. Réactif suivant la revendication 11, caractérisé en ce qu'il contient du tampon glycylglycine ou du tampon tricine, pH 7,0 à 9,0.

18. Réactif suivant la revendication 11, caractérisé en ce qu'il contient en outre un agent tensioactif.

19. Réactif suivant la revendication 11, caractérisé en ce qu'il se présente imprègné sur une matière de support telle que du papier.

FIG. 1

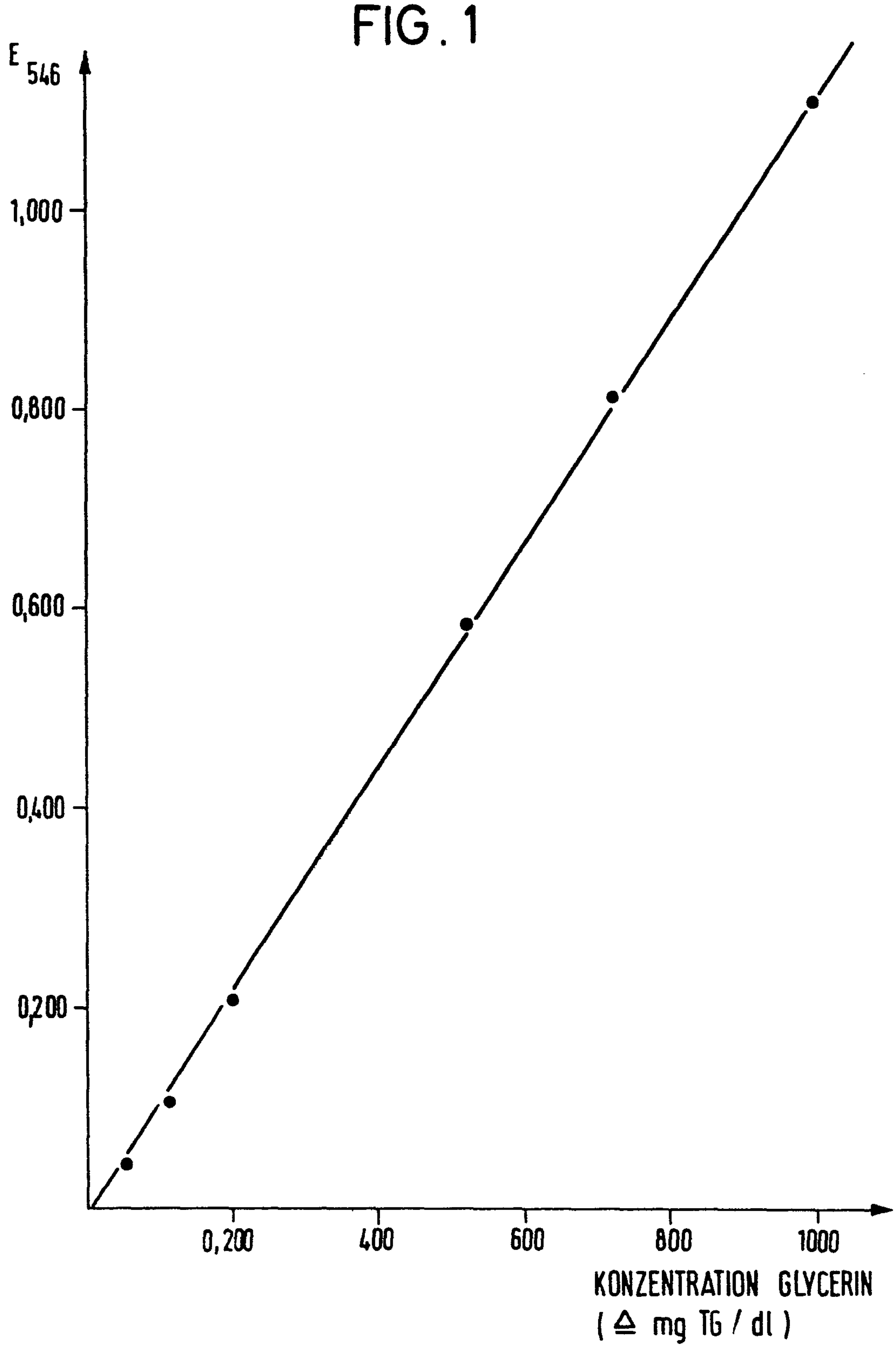
E 546
1,000
0,800
0,600
0,400
0,200
0,200
400
600
800
1000
KONZENTRATION GLYCERIN
(≙ mg TG / dl)

FIG. 2

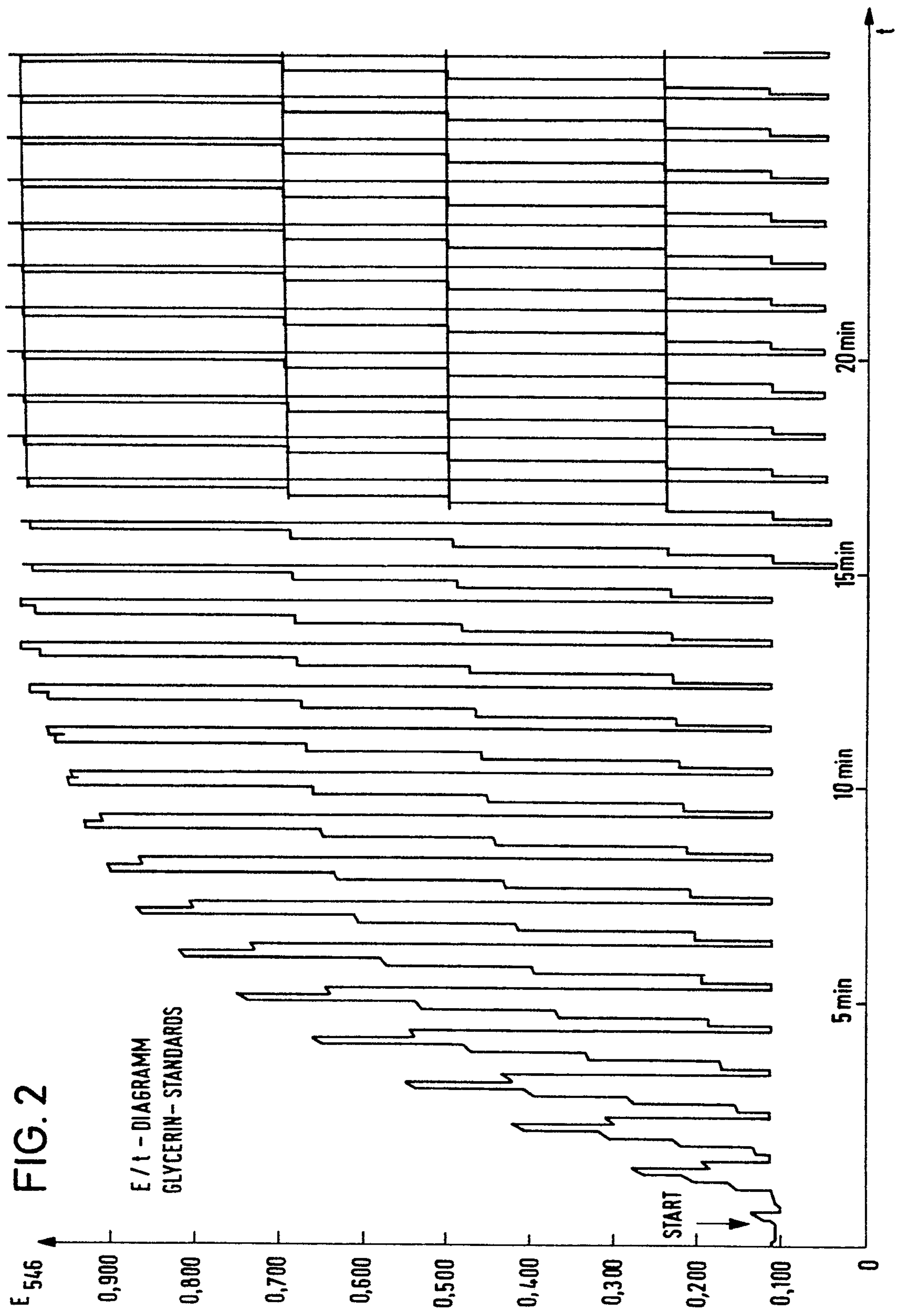
E / t - DIAGRAMM
GLYCERIN - STANDARDS
START
$E_{546}$
0,900
0,800
0,700
0,600
0,500
0,400
0,300
0,200
0,100
0
5 min
10 min
15 min
20 min
t

FIG. 3

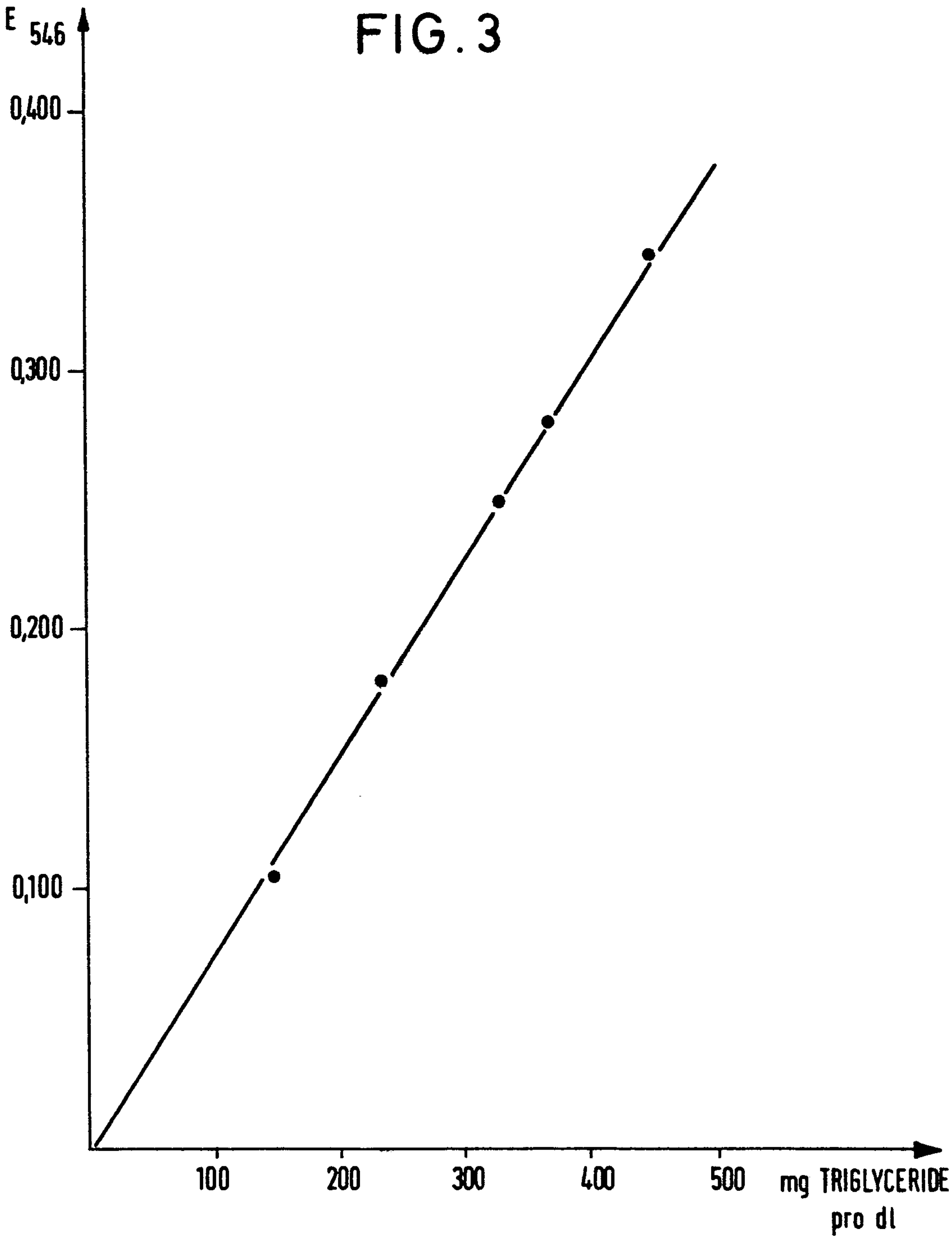
E 546
0,400
0,300
0,200
0,100
100
200
300
400
500
mg TRIGLYCERIDE
pro dl

FIG. 4

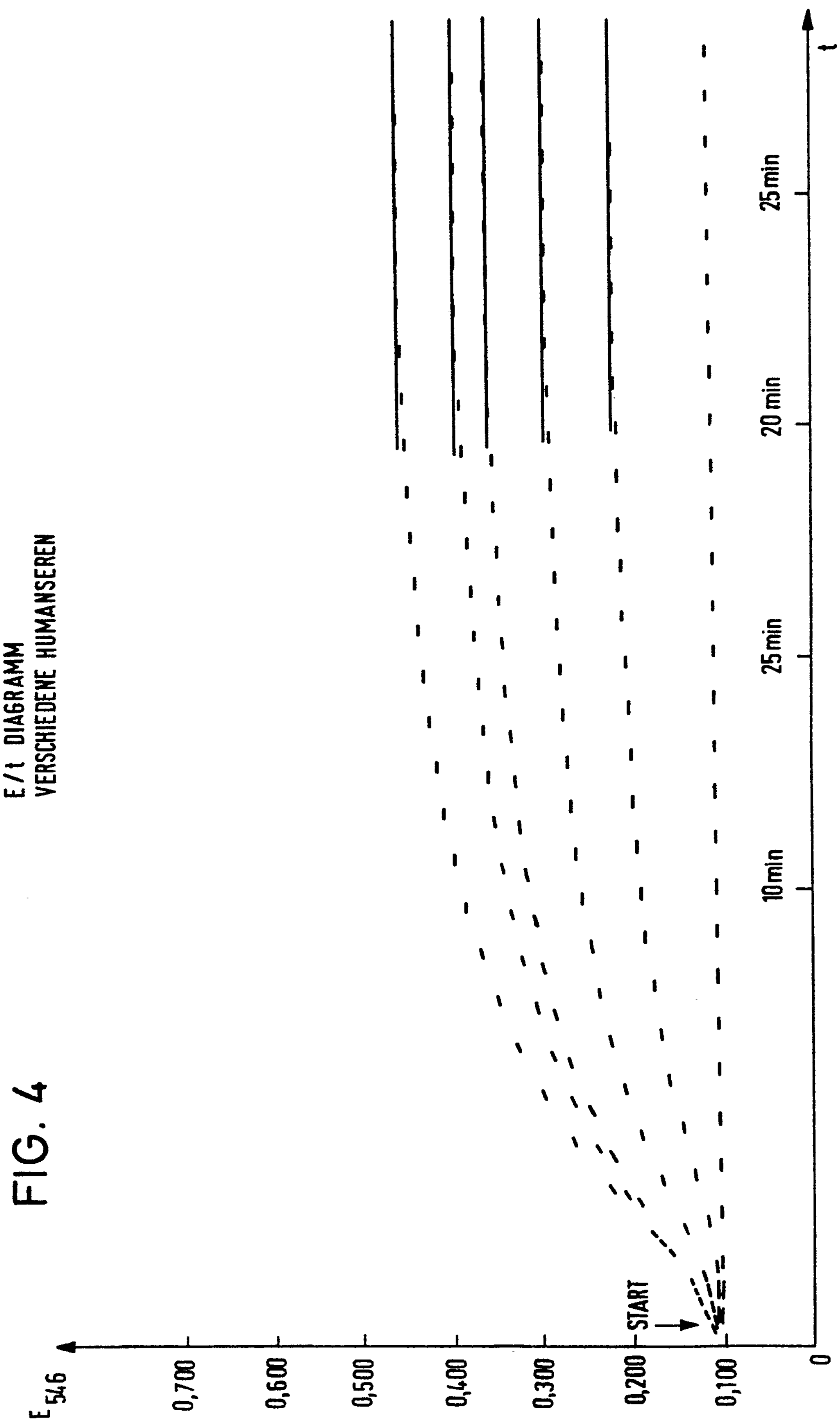
E / t DIAGRAMM
VERSCHIEDENE HUMANSEREN
$E_{546}$
0,700
0,600
0,500
0,400
0,300
0,200
0,100
0
START
10 min
25 min
20 min
25 min
t

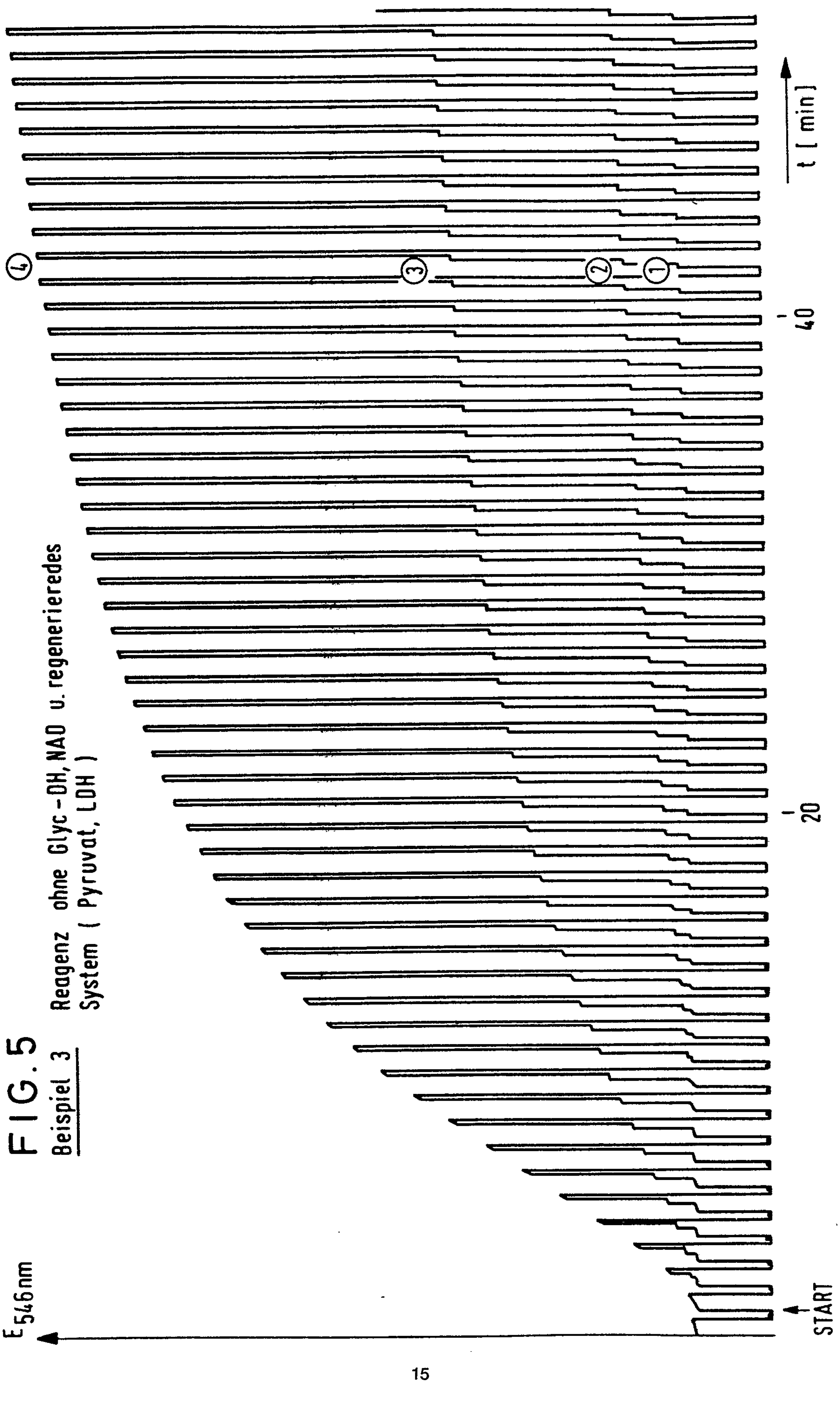
Beispiel 3
Reagenz ohne Glyc-DH, NAD u. regenerieredes
System ( Pyruvat, LDH )
E546nm
START
20
40
t [ min ]
1
2
3
4

FIG. 5

FIG. 6

Beispiel 4 Reagenz ohne Glyc - DH, NAD u. regenierendes System ( Pyruvat, LDH )

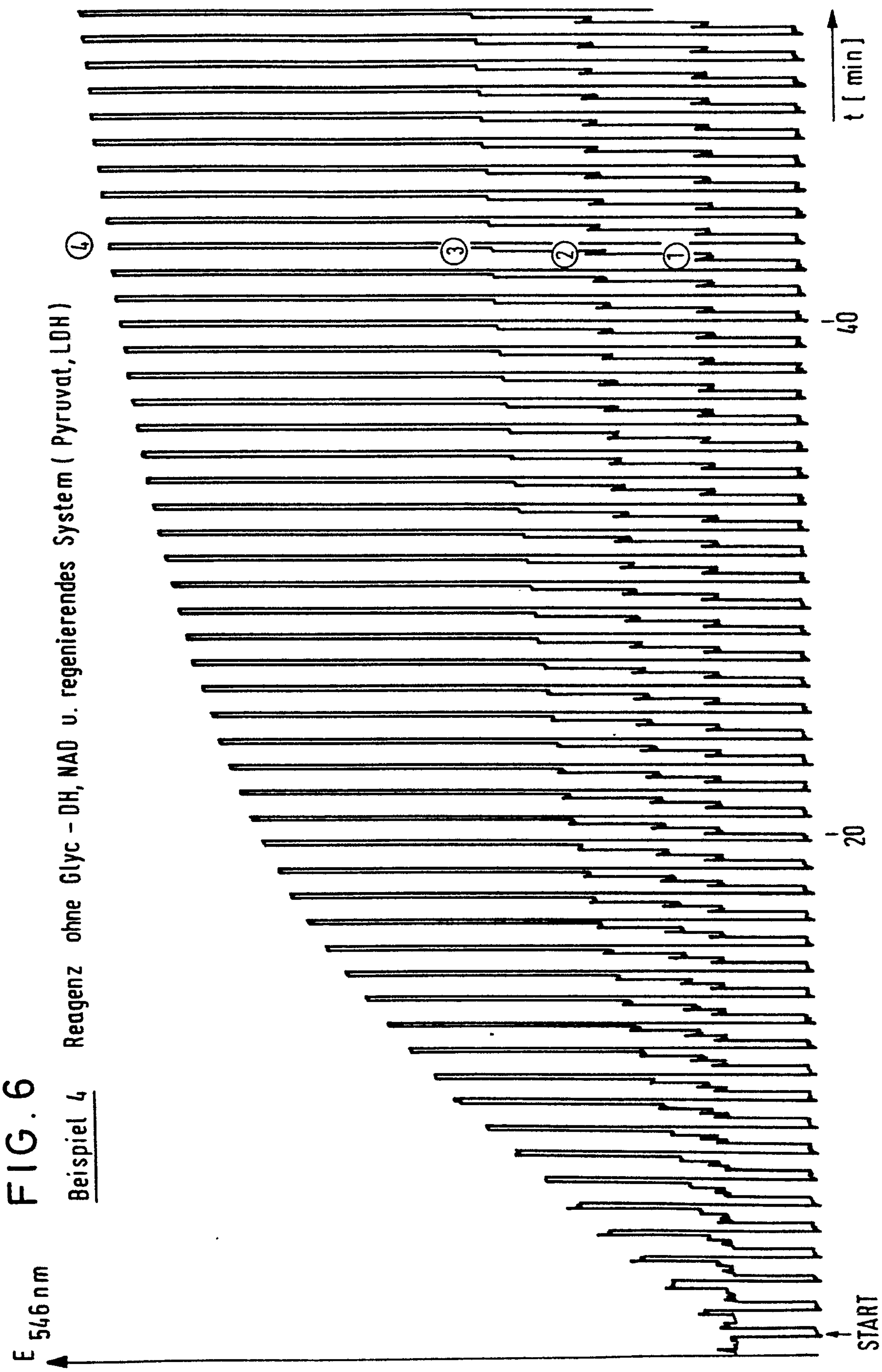

FIG. 7

Beispiel 5  Reagenz ohne regenerierendes System ( Pyruvat, LDH )

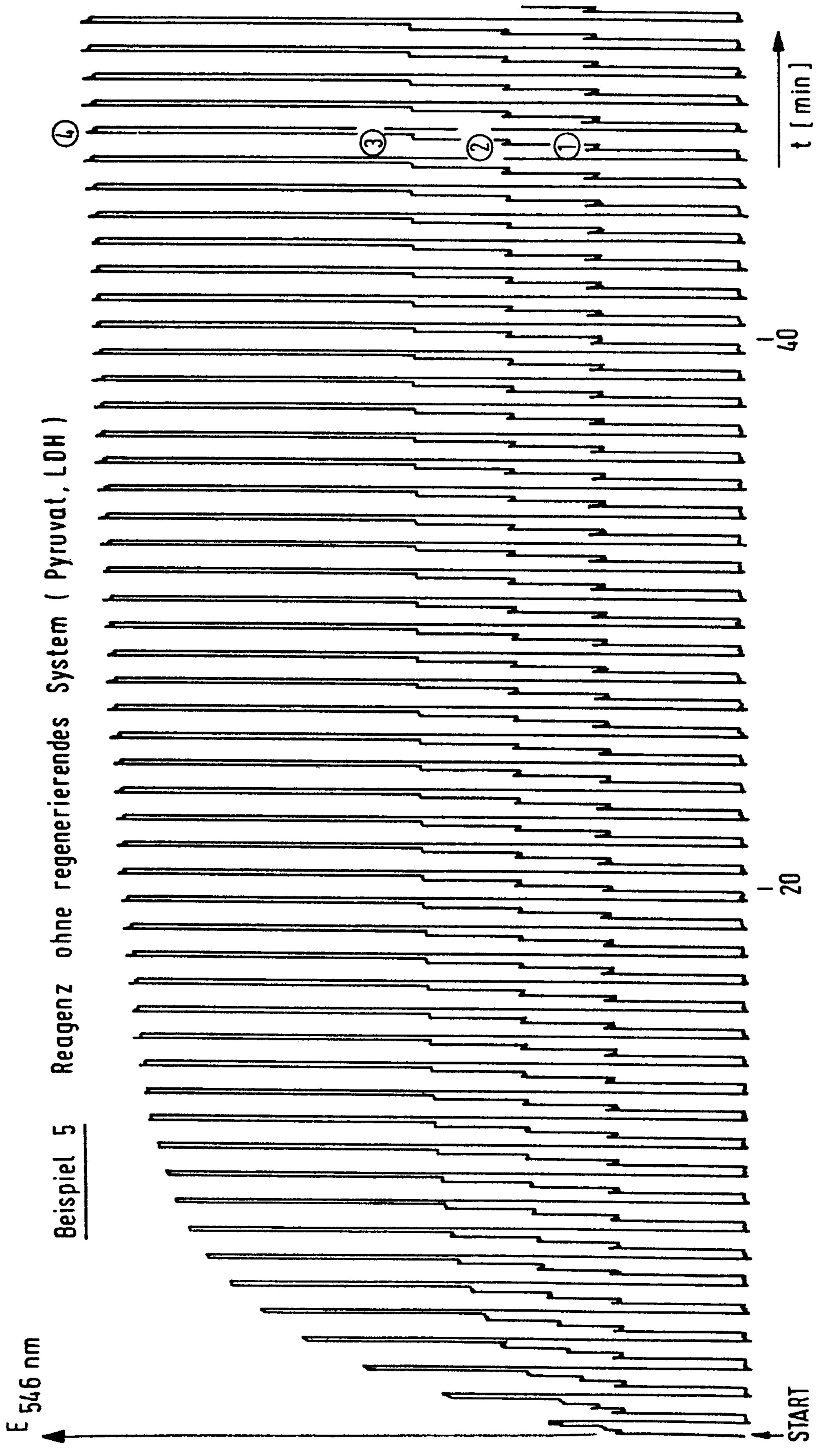

FIG. 8

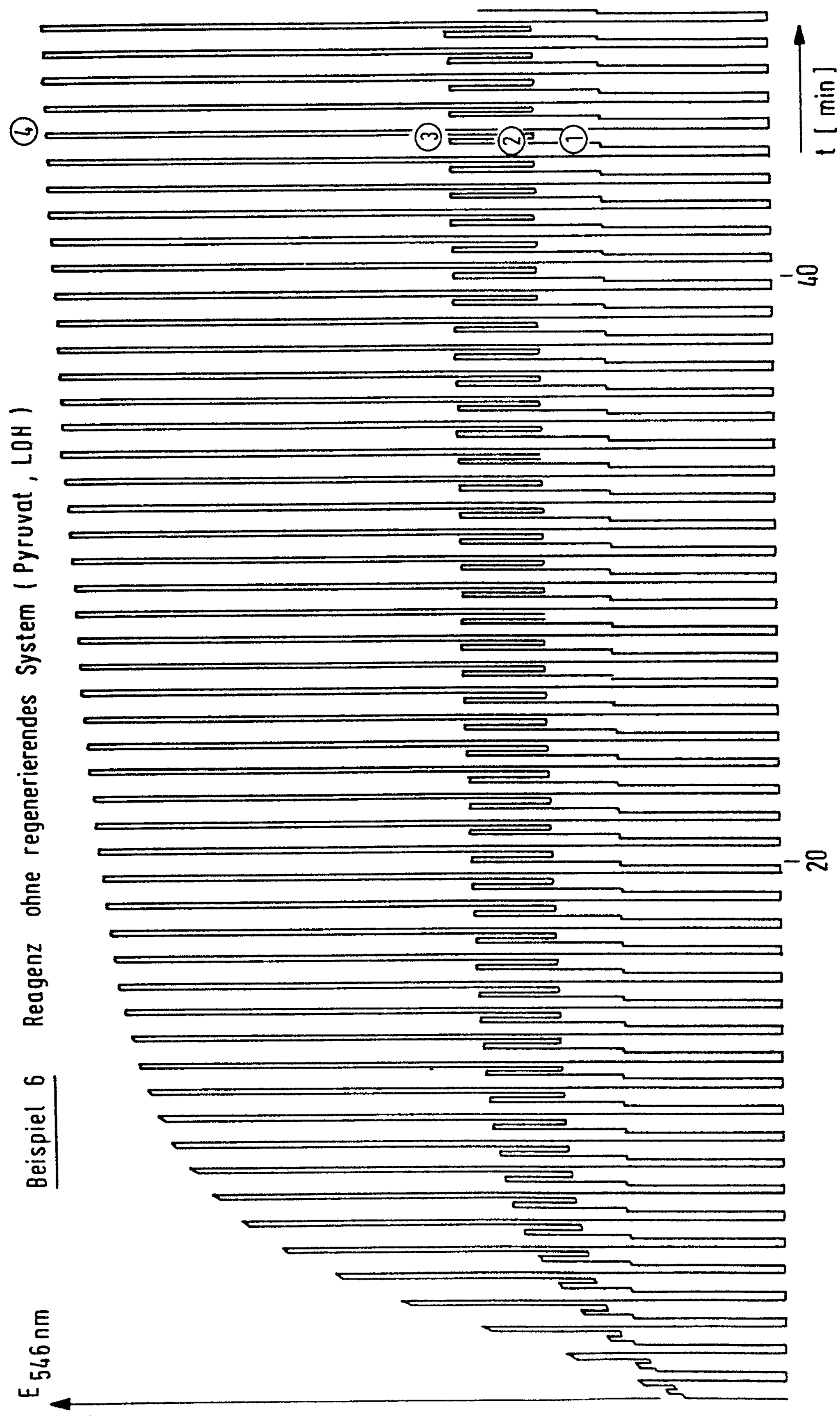

Beispiel 6
Reagenz ohne regenerierendes System ( Pyruvat , LDH )
$E_{546nm}$
t [ min ]
20
40
1
2
3
4

FIG. 9

Beispiel 7 komplettes Reagenz

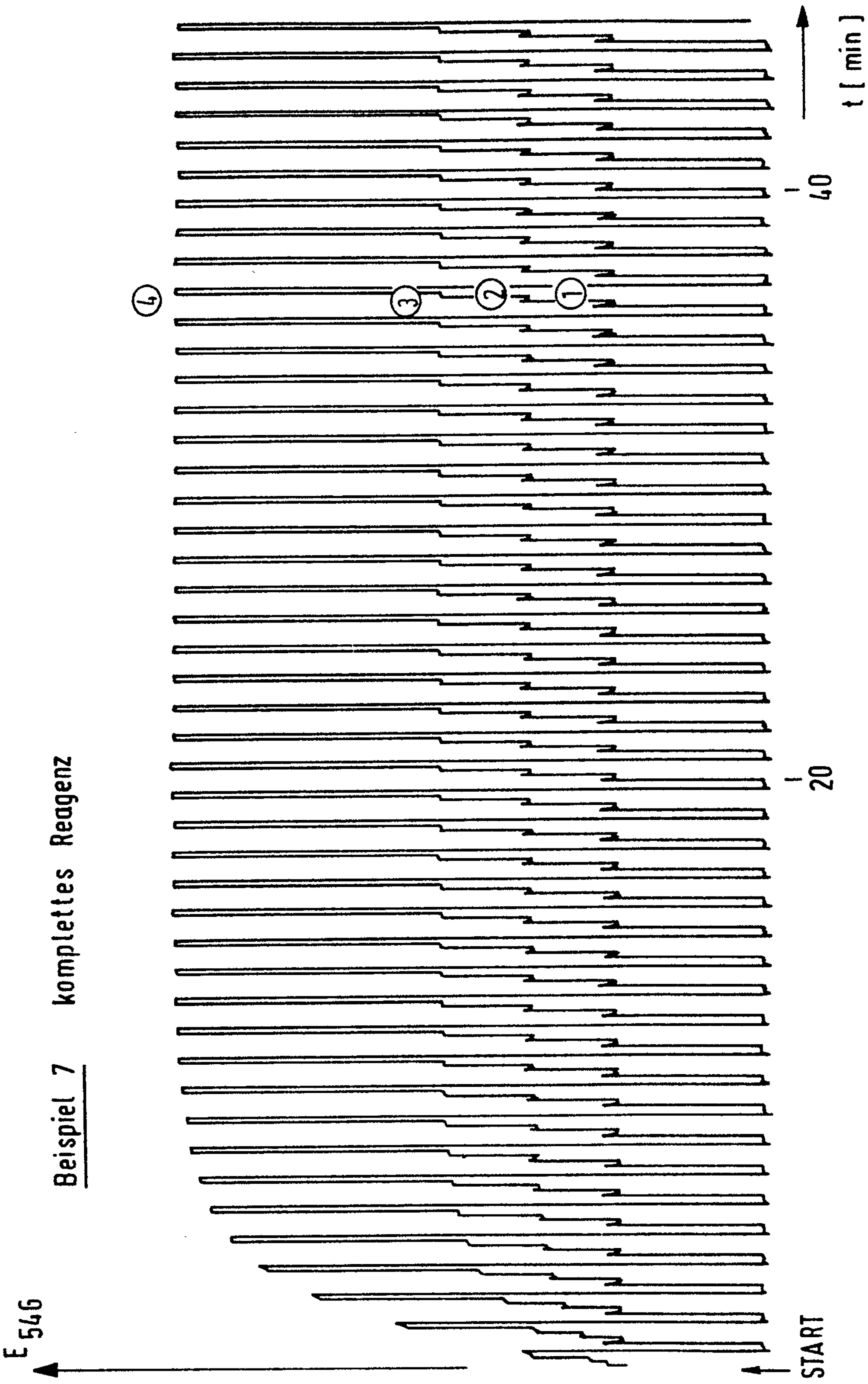

FIG.10